# EUROPEAN PATENT SPECIFICATION

(11) **EP 4 661 808 B1**
(45) Date of publication and mention of the grant of the patent: **30.09.2026**
(21) Application number: 24718931.9
(22) Date of filing: 08.03.2024
(51) Int. Cl.: A61F 2/24, A61B 17/12, A61F 2/06

(54) **FLOW RESTRICTORS FOR BLOOD VESSELS**
DURCHFLUSSBEGRENZER FÜR BLUTGEFÄSSE
RÉDUCTEURS DE DÉBIT POUR VAISSEAUX SANGUINS

(30) Priority: 13.03.2023 US 202363489836 P
(43) Date of publication of application: 17.12.2025
(73) Proprietor: Edwards Lifesciences Corporation, Irvine, CA 92614 (US)
(72) Inventor: SENESH, Gil, 1794000 Adi (IL)
(74) Representative: Venner, Julia Ann
(86) International application number: PCT/US2024/019182
(87) International publication number: WO 2024/191845

(56) References cited:
- US-A1- 2018 036 109
- US-A1- 2018 289 486

## Description

### CROSS-REFERENCE TO RELATED APPLICATIONS

This application claims the priority benefit of U.S. Provisional Patent Application No. 63/489,836, filed on March 13, 2023.

### TECHNICAL FIELD

This disclosure relates generally to the field of medical devices and procedures, and more specifically to the field of blood flow management in blood vessels.

### BACKGROUND

Chronic kidney disease (CKD) is a common comorbidity with many patients who suffer from chronic Heart Failure (HF). HF patients may also have elevated right atrium pressure, which may impair kidney function. In HF patients with elevated right atrium pressure, the kidneys may attempt to perform a diuresis process, but such a process may be difficult to perform efficiently due to the elevated pressure. For example, elevated right atrium pressure may hinder the ability of the kidneys to drive forward the flow of blood for accomplishing proper and efficient diuresis. Such unbalanced pressure coupled with the typical poor kidney efficiency of CKD patients may lead to an unending cycle of fluid overload for a person, which may result in an increase in congestion and heart failure admissions to the hospital.

US 2018/289486 describes a valve prosthetic implant for treating a vein or other blood vessel which includes a tubular, expandable anchoring frame extending from a proximal end to a distal end of the implant, a valve seat formed at or near the middle of the anchoring frame, an expandable ball disposed within the lumen of the anchoring frame, and a ball retention tether attached to the expandable ball and to the valve seat and/or the anchoring frame. The anchoring frame may include a cylindrical proximal portion at the proximal end, a cylindrical distal portion at the distal end, an inwardly angled inlet portion between the cylindrical proximal portion and a middle of the anchoring frame, and an inwardly angled outlet portion between the cylindrical distal portion and the middle of the anchoring frame.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention

Described herein are one or more methods and/or devices to facilitate management of blood flow through and/or into one or more blood vessels and/or chambers of a heart. There is a need for new and useful systems and methods for flow restrictors for blood vessels In particular, there is a need for systems, devices, and methods that enable modulating and/or balancing of blood flow through a blood vessel, for example, to occlude, partially occlude, and/or otherwise manage or regulate blood flow to or through a portion of a blood vessel.

In some embodiments, a flow restrictor for a blood vessel, comprises: a frame positionable within a blood vessel; an inner cell; a leaflet; and a lumen through the flow restrictor, wherein the lumen is defined at least in part by the leaflet. The frame can comprise an outer cell comprising an inflow end and an outflow end. The inner cell can comprise an anchoring junction coupled to the outflow end of the outer cell, and a movable junction. The leaflet can comprise an inflow edge and an outflow edge, wherein the inflow edge is coupled to the movable junction of the inner cell, and the outflow edge is coupled to the anchoring junction. The movable junction of the inner cell and the inflow edge of the leaflet can be configured to move axially in response to a first increase in a blood pressure within the blood vessel, and in response to the blood pressure within the blood vessel being within a first blood pressure range. The leaflet can be curved and can be configured to bend in response to the movable junction moving axially. The movable junction and the leaflet can be configured such that the lumen changes size when the movable junction moves.

### BRIEF DESCRIPTION OF THE DRAWINGS

The foregoing is a summary, and thus, necessarily limited in detail. The above-mentioned aspects, as well as other aspects, features, and advantages of the present technology are described below in connection with various embodiments, with reference made to the accompanying drawings.
FIGs. 1A and 1B show schematics of examples of portions of a flow restrictor for a blood vessel, in accordance with some embodiments.
FIGs. 2A-2C show schematics of an example of portions of a flow restrictor for a blood vessel including an example of a leaflet shown in a side view, in accordance with some embodiments.
FIGs. 3A-3C show schematics of an example of portions of a flow restrictor from top-down views showing a lumen formed (at least in part) by leaflets, in accordance with some embodiments.
FIGs. 4A-4C show schematics of an example of portions of a flow restrictor including an example of a leaflet shown such that outside surface and the inflow edge of the leaflet are visible, in accordance with some embodiments.
FIGs. 5A-5C show schematics of an example of portions of a flow restrictor, which are similar to the flow restrictors in FIGs. 4A-4C, and include examples of flow restrictors with three leaflets, in accordance with some embodiments.
FIGs. 6A-6D show examples of leaflet shapes for flow restrictors, in accordance with some embodiments.
FIG. 7 shows an example of a method related to restricting blood flow within a blood vessel, in accordance with some embodiments.
FIG. 8 shows an example of a method related to restricting blood flow within a blood vessel, in accordance with some embodiments.
FIG. 9 shows a schematic representation of portions of a subject with any of the flow modulating devices described herein.

### DETAILED DESCRIPTION

The foregoing is a summary, and thus, necessarily limited in detail. The above-mentioned aspects, as well as other aspects, features, and advantages of the present technology will now be described in connection with various embodiments. The inclusion of the following embodiments is not intended to limit the disclosure to these embodiments, but rather to enable any person skilled in the art to make and use the contemplated invention(s). Other embodiments may be utilized, and modifications may be made without departing from the spirit or scope of the subject matter presented herein. Aspects of the disclosure, as described and illustrated herein, can be arranged, combined, modified, and designed in a variety of different formulations, all of which are explicitly contemplated and form part of this disclosure.

In general, the systems and methods described herein may enable modulating and/or balancing of blood flow through a blood vessel. The modulating and/or balancing of blood flow may be performed by the devices described herein to occlude, partially occlude, and/or otherwise manage or regulate blood flow to or through a portion of a blood vessel. In some examples, such modulation and/or balancing of blood flow to or through a blood vessel may result in additionally modulating pressure in the right atrium of the heart and/or other organs of the body.

The examples presented herein may relate to providing devices, methods, and/or methods of treatment (MOTs) for modulating and/or otherwise managing blood flow to or through particular blood vessels. The terminology of restricting blood flow, regulating blood flow, modulating blood flow, managing blood flow, and balancing blood flow may include causing regulation of blood pressure, modulation of blood pressure, management of blood pressure, and/or balancing of blood pressure. As such, for example, a flow modulation device is synonymous with a pressure regulating device (i.e., a flow regulator is synonymous with a pressure regulator). In some examples, the devices described herein may include blood flow management devices for reducing blood flow through a blood vessel, such as the Superior Vena Cava (SVC), the Inferior Vena Cava (IVC) and/or related vessels. Managing blood flow through the SVC or IVC can be achieved by the devices described herein to provide an advantage of improving perfusion of the kidneys. In particular, the devices described herein may enhance a pressure gradient across the kidneys by decreasing central venous pressure by restricting, balancing, or otherwise modifying particular blood flow through the SVC and/or IVC, resulting in improved kidney perfusion and function.

In some examples, the devices, methods, and/or MOTs described herein may be utilized to solve a technical problem of unwanted pressure increases in the right atrium in patients that have chronic kidney disease (CKD) and/or heart failure (HF). For example, patients with CKD and/or HF may exhibit reduced kidney function when pressure in the right atrium of the heart is above a predefined pressure threshold. The predefined pressure threshold may be used as a basis to determine whether a patient is exhibiting low vessel pressure (e.g., below the predefined pressure threshold) or high vessel pressure (e.g., above the predefined pressure threshold). When vessel pressure is determined to be high, the devices, methods, and/or MOTs can provide a technical solution to the technical problem recited above. For example, each of the devices described herein may be used to decrease pressure within one or more vessels to avoid right atrium pressure increases and/or pressure variations. In particular, the devices, methods, and/or MOTs described herein can be used to reduce and/or maintain low pressure in the right atrium, which provides a technical effect of enabling the kidneys to more effectively filter blood.

In addition, the devices, methods, and/or MOTs described herein can solve a further technical problem of accumulation of blood in the venous system. For example, the devices described herein may be used to reduce the accumulation of blood in the venous system, which can provide an advantage and technical effect of ensuring that pressure is not increased in the IVC. Such devices can advantageously eliminate excessive hospital readmissions and/or can provide for a long-term blood flow management therapy, improving both quality of life and overall survival rates and with a lower cost to a healthcare system.

Furthermore, the devices, methods, and/or MOTs described herein can be used to solve a further technical problem of regulating blood flow return, thus further mitigating pressure build-up in the right atrium. The examples described herein can perform blood flow management actively and/or passively to assist in reducing and/or maintaining right atrium pressures to a relatively low pressure even when a surge in blood volume occurs in one or more vessels of the venous system.

In some examples, the devices, methods, and/or MOTs described herein can be used to solve a further technical problem of exertion-related blood pressure in patients that have a flow restrictor implanted within a blood vessel. For example, flow restrictor devices described herein can restrict blood flow through a blood vessel at elevated blood pressures (e.g., from about 10 mmHg to about 25 mmHg, or from about 15 mmHg to about 25 mmHg), and also advantageously permit a larger amount of blood flow (by not restricting or minimally restricting blood flow through the blood vessel) at exertion-related blood pressures (e.g., greater than 25 mmHg, or greater than 30 mmHg). In some cases, the flow restrictors described herein can permit a larger amount of blood flow through the blood vessel at exertion-related blood pressures (e.g., greater than 25 mmHg, or greater than 30 mmHg), which can advantageously provide a patient with sufficient blood flow through the blood vessel during periods of exercise or stress to prevent negative side effects (e.g., fainting).

In some cases, patients who suffer from congestive heart failure (CHF) can also experience impaired renal function. Impaired renal function can be caused by increased systemic venous congestion as a result of low cardiac output and low blood pressure. The renal pressure gradient (between the renal arteries and renal veins) may be decreased due to elevated renal venous pressure, lowering glomerular filtration rate (GFR). GFR is the rate at which the kidney filters blood, for example, below 90 mL/min, which can be indicative of chronic kidney disease (CKD) that may eventually lead to end stage renal failure. Thus, reduction of renal venous pressure may improve GFR and reduce blood volume retention. Nevertheless, it may be desirable for a device (e.g., a flow restrictor described herein) for limiting central venous volume to operate in a bimodal fashion, to reduce venous pressure when the patient is at rest (e.g., under normal or elevated blood pressure ranges), yet allow undisturbed or minimally disturbed venous flow when the patient exercises (or experiences an exertion-related blood pressure), so as to meet the dynamic flow/pressure requirements. Moreover, any such solution, when provided as an implantable device, can be percutaneously deliverable and can operate in a manner that minimizes risk of thrombosis.

Disclosed herein are systems and methods for a flow restrictor for a blood vessel. In some examples, the implantable flow modulating devices (or flow restrictors) described herein may be used in blood flow occlusion therapy. For example, the devices described herein may relate to venous occlusion therapy using implantable and/or electronically controlled flow restricting devices for the treatment of acute heart failure. Some devices may be non-implantable or partially implantable. Some devices may operate without any powered input and are triggered by changes in anatomy and/or changes in physiology (e.g., venous pressure in the IVC or SVC).

### SYSTEMS AND DEVICES

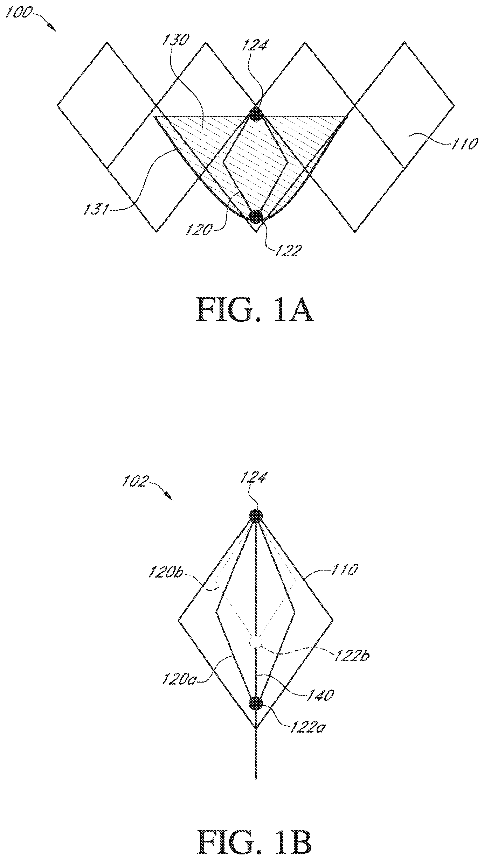

FIGs. 1A-6D show examples of flow modulating devices that are flow restrictors for blood vessels. The flow restrictors described herein can be used to occlude, partially occlude, and/or otherwise modulate, manage, or regulate blood flow to or through a portion of a blood vessel. For example, the flow restrictors described herein may be used to reduce blood pressure in blood vessels and/or organs (e.g., kidneys) downstream from the flow restrictor. The flow restrictors described herein can additionally, or alternatively, be used for any suitable applications, clinical or otherwise. For example, the flow restrictors described herein can be configured and/or adapted to function for any suitable application requiring restriction of blood flow within a blood vessel, for example, to increase blood flow through an organ (e.g., the kidneys), to modulate pressure in the right atrium of the heart, and/or to reduce the accumulation of blood in the venous system.

In some examples, flow restrictors for a blood vessel include one or more leaflets coupled to one or more movable junctions that cause the one or more leaflets to bend (or flex or deform) in response to (and in some cases proportional to) elevated blood pressure (e.g., venous pressure). For example, excessive blood volume in the blood vessel, or volume overload, can lead to elevated blood pressure conditions. A flow restrictor defines a lumen, the size of which may be modulated or further defined at least in part by the one or more leaflets. The bending of the one or more leaflets causes the lumen to change size, thereby changing the restriction of blood flow through the lumen of the flow restrictor and thus through the blood vessel. In some cases, the movable junctions can be actively moved (or actuated), for example, in response to a sensor detecting a change in blood pressure. In other cases, the movable junctions can be passively moved (or actuated), for example, in response to changes in blood pressure applying pressure to one or more surfaces of the one or more leaflets. These flow restrictors can provide a mechanically driven solution by which GFR (a metric of kidney function) can be improved in CKD patients, as an alternative (or in lieu of) conventional pharmaceutical treatments.

Although numerical or alphabetical designations or sequence designations (first, second, third, etc.) are used herein, these shall not be construed as connoting a sequence of events or steps. Any steps or events can have any configuration or sequence.

FIG. 1A shows a schematic of an example of a flattened portion of a flow restrictor 100 for a blood vessel, including a frame 110, an inner cell 120, and a simplified example of a leaflet (or flap) 130. Inner cell 120 is coupled to the frame at anchoring junction 124, and inner cell 120 also has a movable junction 122. Movable junction 122 can move using active or passive means, as described herein. Leaflet (or flap) 130 is coupled to the inner cell 120 via movable junction 122 and anchoring junction 124 such that the leaflet moves in response to movable junction 122 moving. An inflow end 131 of leaflet (or flap) 130 that has a scalloped shape is shown in FIG. 1A. Leaflets are further described herein and shown in subsequent figures.

FIG. 1B shows a schematic of an example of a portion of a flow restrictor 102 for a blood vessel, including a portion of a frame 110, an inner cell 120a, 120b (indicating different positions of inner cell), and a control element 140. Inner cell 120a is shown at a first position, where movable junction 122a is at a first position. Inner cell 120b is shown in a second position (shown in dashed lines), where movable junction 122b has moved to a second position thereby causing the shape of inner cell 120a, 120b to change (or deform or bend). Control element 140 enables movable junction 122a, 122b (indicating different positions of movable junction) to move. For example, control element 140 can be a shape memory material (e.g., Nitinol^{®}) which can change length or shape when an electrical current is applied, thereby causing movable junction 122a, 122b (indicating different positions of movable junction) to move. Other active mechanisms and systems described herein can also be used to move movable junction 122a, 122b and/or control element 140.

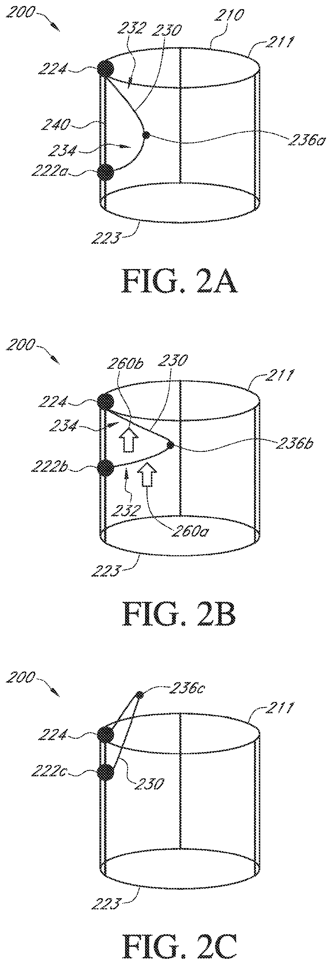

FIGs. 2A-2C show various schematics of an example of portions of a flow restrictor 200 which includes a simplified example of a leaflet 230 shown in a side view to illustrate the curvature of the leaflet caused by moving the inflow edge of the leaflet. Leaflet 230 is made from a flexible (or bendable, or deformable) material, and is coupled to movable junction 222a (at a first position), movable junction 222b (at a second position), movable junction 222c (at a third position) and anchoring junction 224. Leaflet 230 is shown as a line in FIGs. 2A-2C, but in some cases, leaflet 230 may be curved in more than one dimension such that the line depicting leaflet 230 depicts a cross-section of leaflet 230, in some cases, as shown in subsequent figures. Leaflet 230 has an inside surface 234 and an outside surface 232. Control element 240 is similar to control element 140 in FIG. 1B and is actuated (passive or actively) to move movable junction 222a, 222b, 222c. Control element 240 and anchoring junction 224 can be coupled to frame 210. Frame 210 is shown as a cylinder, but can be other shapes in other examples, such as an ovular prism, or a lattice structure (such as that shown in FIG. 1A). The inflow edge of leaflet 230 is coupled to the movable junction 222a, 222b, 222c and the inflow edge of the leaflet 230 moves as the movable junction 222a, 222b, 222c moves, thereby causing the leaflet 230 to bend (or flex or deform) as shown in FIGs. 2A-2C. FIG. 2A shows movable junction 222a in a first position, proximal to an inflow end 223 of frame 210 and distal or removed from anchoring junction 224. FIG. 2B shows movable junction 222b in a second position, closer to anchoring junction 224 than movable junction 222a at the first position. FIG. 2C shows movable junction 222c in a third position, proximal to anchoring junction 224 and closer to anchoring junction 224 than movable junction 222b at the second position.

FIG. 2B shows that blood flow can apply pressure 260a to the outside surface 232 of leaflet 230 or can apply pressure 260b to the inside surface 234 of leaflet 230. The amount of pressure applied to the outside surface 232 compared to the inside surface 234 can be determined by a shape of the leaflet 230, including a shape of the inflow edge. For example, a wide inflow edge may block most of the blood flow and cause at least some or a majority of the pressure to be applied to the outside surface 232 of the leaflet 230. A narrower inflow edge can block less of the blood flow and cause less of the pressure to be applied to the outside surface 232 of the leaflet 230 and more of the pressure to be applied to the inside surface 234 of the leaflet 230. In some passive embodiments, the pressure 260a and/or pressure 260b applied by the blood flow can cause the movable junction 222a, 222b, 222c to move. For example, higher blood pressure can cause the movable junction 222a, 222b, 222c to move towards the outflow end 211 of flow restrictor 200.

In some embodiments, flow restrictor 200 in FIGs. 2A-2C can be actively actuated. For example, as shown in FIG. 2A, control element 240 can be a shape memory material that is actively actuated (e.g., in response to a change in blood pressure), and movable junction 222a, 222b, 222c can be moved by control element 240. In some cases, one or more sensors may measure a blood pressure in a blood vessel, and the flow restrictor may be actively actuated based on signals from the one or more sensors. In some cases, control element 240 may be coupled to an inner cell (not shown in FIGs. 2A-2C, but as shown in FIG. 1B), and anchoring junction 224 may be coupled to the inner cell and the frame 210. In such cases, the inner cell can be coupled to the movable junction 222a, 222b, 222c and the movement of the movable junction 222a, 222b, 222c causes the inner cell to move (or bend or deform, e.g., as shown in FIG. 1B), thereby causing the leaflet 230 to move (or bend or deform) as shown in FIGs. 2A-2C.

Flow restrictor 200 shown in FIGs. 2A-2C is a bimodal flow restrictor with leaflet configurations designed to restrict blood flow in response to elevated blood pressure. In some cases, the bimodal flow restrictor shown in FIGs. 2A-2C restricts blood flow in proportion to elevated blood pressure during normal activity of the patient (e.g., as shown in FIG. 2B), and allows blood to flow therethrough with minimal obstruction during exertion-related elevated pressure (e.g., due to exercise activity and/or stress, as shown in FIG. 2C). In some cases, the bimodal flow restrictor shown in FIGs. 2A-2C restricts blood flow in proportion to elevated blood pressure in a first blood pressure range (e.g., from about 10 mmHg to about 25 mmHg, or from about 15 mmHg to about 25 mmHg) and allows blood to flow therethrough with minimal obstruction in a second blood pressure range. The second blood pressure range can be greater than a threshold blood pressure (e.g., about 25 mmHg or about 30 mmHg).

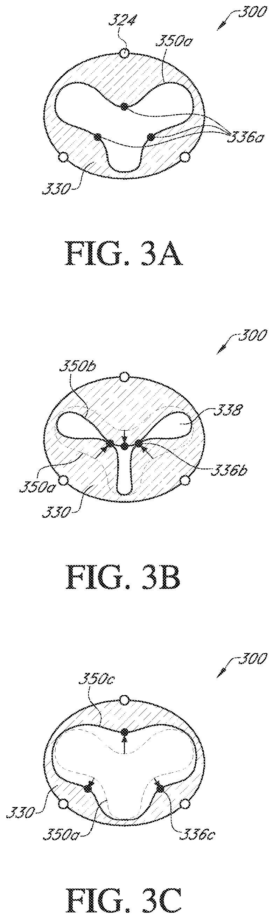

FIGs. 3A-3C show various schematics of an example of portions of a bimodal flow restrictor 300 from top-down views, showing a lumen 350a, 350b, 350c defined (at least in part) by leaflets 330. The leaflets 330 are formed from coupling three leaflets together, where each leaflet being coupled together in this example is similar to leaflet 230 in FIGs. 2A-2C. FIGs. 3A-3C are described in combination with FIGs. 2A-2C to illustrate shared features between the figures and to highlight differences between the figures.

FIG. 2A shows bimodal flow restrictor 200 under normal blood pressure conditions (e.g., about 1 mmHg to about 15 mmHg), where leaflet 230 has an outer point 236a. FIG. 3A shows a bimodal flow restrictor 300 under normal blood pressure conditions (e.g., about 1 mmHg to about 15 mmHg), where opposing sides of leaflet 330 are spaced apart such that the outer points 336a (approximately corresponding to point 236a in FIG. 2A) are, for example, about 60% to about 80% closed (meaning that the lumen 350a formed from leaflet 330 comprises about 40% to about 20% of the total cross-sectional area of the blood vessel, at the location of the flow restrictor under normal blood pressure conditions). Opposing sides of leaflet 330 can be spaced apart such that the outer points 336a are from about 20% to about 80% closed (meaning that the lumen 350a comprises about 80% to about 20% of the total cross-sectional area of the blood vessel, at the location of the flow restrictor, under normal blood pressure conditions).

FIG. 2B shows bimodal flow restrictor 200 under elevated blood pressure conditions, where the blood pressure in the blood vessel in FIG. 2B is higher than the blood pressure in the blood vessel in FIG. 2A (e.g., from about 10 mmHg to about 15 mmHg, or from about 15 mmHg to about 25 mmHg). For example, excessive blood volume in the blood vessel, or volume overload, can lead to such elevated blood pressure conditions. Movable junction 222b has moved towards the outflow end 211 of the flow restrictor 200, thereby causing leaflet 230 to bend (or flex or deform) such that it has an outer point 236b. FIG. 3B shows a bimodal flow restrictor 300 under elevated blood pressure conditions (e.g., from about 10 mmHg to about 15 mmHg, or from about 15 mmHg to about 25 mmHg), where opposing sides of leaflet 330 are spaced closer together such that the outer points 336b (approximately corresponding to point 236b in FIG. 2B) are, for example, about 90% to about 100% closed (meaning that the lumen 350a formed from leaflet 330 comprises about 10% to about 0% of the total cross-sectional area of the blood vessel, at the location of the flow restrictor under elevated blood pressure conditions). As shown in FIG. 3B, the leaflets 330 may bend (or flex or deform) and cause blood to flow through gaps (e.g., gap 338) formed in the flow restrictor 300. In other cases, leaflets 330 may bend (or flex or deform) such that few or restricted or no gaps (e.g., gap 338) are formed. In some embodiments, leaflets 330 may bend (or flex or deform) such that gaps that are formed are larger or smaller, or gaps are formed in different locations than the gaps (e.g., gap 338) shown in FIG. 3B. In general, under elevated blood pressure conditions, opposing sides of leaflet 330 can be spaced closer together (or touching or interacting) such that outer points 336b are about 60% to about 100% closed (meaning that the lumen 350b comprises about 40% to about 0% of the total cross-sectional area of the blood vessel, at the location of the flow restrictor, under the elevated blood pressure conditions). Lumen 330b closing in FIG. 3B reduces the amount of blood that can flow through the flow restrictor in this example, thereby lowering blood pressure downstream from leaflet 330.

FIG. 2C shows bimodal flow restrictor 200 under exertion-related blood pressure conditions, where the blood pressure in the blood vessel in FIG. 2C is higher than the blood pressure in the blood vessel in FIGs. 2A and 2B (e.g., greater than about 25 mmHg, or greater than about 30 mmHg). Movable junction 222c has moved towards the outflow end 211 of the flow restrictor even farther than in FIG. 2B, thereby causing leaflet 230 to bend (or flex or deform) such that it prolapses (wherein leaflet 230 moves in the downstream direction) and has an outer point 236c. FIG. 3C shows the bimodal flow restrictor 300 under exertion-related blood pressure conditions, where the blood pressure in the blood vessel in FIG. 3C is higher than the blood pressure in the blood vessel in FIGs. 3A-3B (e.g., greater than about 25 mmHg, or greater than about 30 mmHg). Leaflet 330 in FIG. 3C is prolapsed (similar to that shown in FIG. 2C) such that opposing sides of leaflet 330 and points 336c are, for example, about 80% to about 100% open (meaning that the lumen 350c comprises about 80% to about 100% of the total cross-sectional area of the blood vessel under the exertion-related blood pressure conditions). In general, under exertion-related blood pressure conditions, leaflet 330 is prolapsed (e.g., leaflet 330 moves in the downstream direction) such that opposing sides of leaflet 330 can be spaced farther apart and outer points 336b are about 5% to about 100% (or about 10% to about 90%) open (meaning that lumen 350c comprises about 5% to about 100% of the total cross-sectional area of the blood vessel, at the location of the flow restrictor, under the exertion-related blood pressure conditions).

In some embodiments, the rigidity of leaflets 230, 330 compared to the coupling regions (e.g., 224 and 222a-c in FIGs. 2A-2C) in the examples shown in FIGs. 2A-3C allow them to prolapse (or collapse downstream) and allow blood flow with minimal restriction due to the exertion-related blood pressure. After the exertion-related blood pressure subsides, and the blood pressure returns to the normal blood pressure conditions or the elevated blood pressure conditions, then leaflets 230, 330 can revert to the configurations shown in FIGs. 2A and 3A or 2B and 3B, respectively.

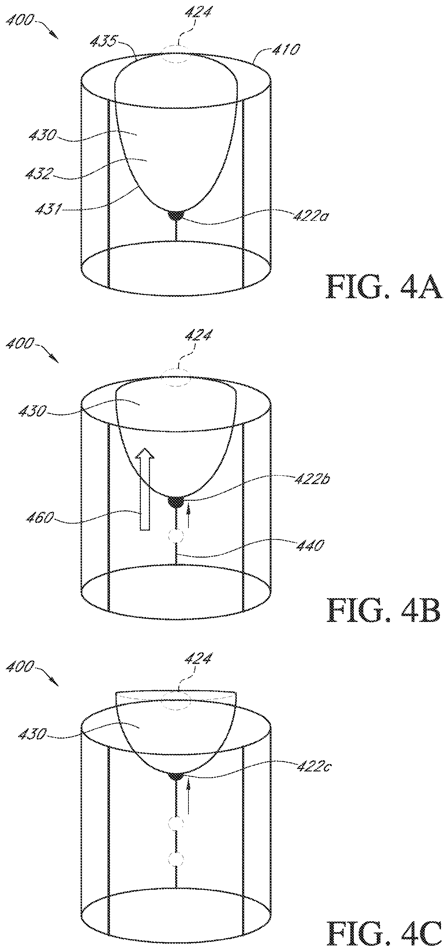

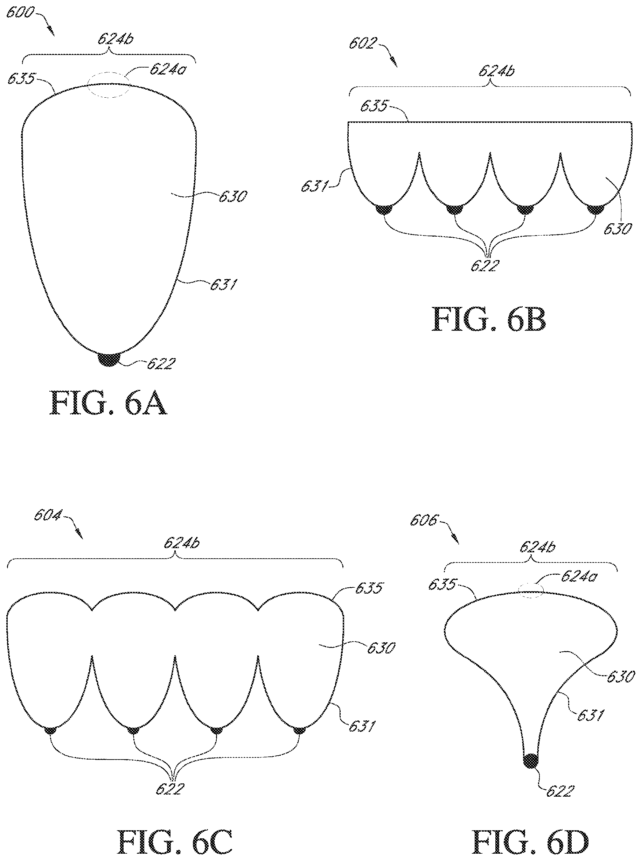

FIGs. 4A-4C show schematics of examples of portions of flow restrictor 400, which includes an example of a leaflet 430. Leaflet 430 is made from flexible (or bendable or deformable) material. Leaflet 430 can be made of, formed or, or comprise metal and/or polymer structures with or without a thermoplastic polyurethane coating, a biological material (bovine/porcine pericardium), or other suitable material. Leaflet 430 has an inflow edge 431 coupled to movable junction 422a, 422b, 422c (depending on leaflet position), and an outflow edge 435 coupled to anchoring junction 424. Leaflet 430 is bent (or flexed or deformed) as shown such that outside surface 432, and the inflow edge 431 of the leaflet are visible. FIG. 6A shows an example of leaflet 430 when flattened onto a plane. Flow restrictor 400 in FIGs. 4A-4C is similar to, and has similar components as, flow restrictor 200 in FIGs. 2A-2C. Anchoring junction 424 is coupled to leaflet 430 and is further coupled to frame 410. Frame 410 is shown as a cylinder, but can be other shapes in other examples, such as an ovular prism, or a lattice structure (such as that shown in FIG. 1A). Anchoring junction 424 can be coupled to leaflet 430 at a point or can be coupled at an area along the outflow edge 435 of leaflet 430. The leaflet 430 is bent in this example (similar to the shape of leaflet 230 in FIG. 2A), such that blood flow 460 applies pressure to the outside surface 432 of leaflet 430 (similar to pressure 260a in FIG. 2B). The inflow edge 431 is scallop shaped (and is relatively wide) in this example, which causes the blood flow to apply pressure to the outside surface 432 of the leaflet 430; however, the blood flow may also apply some pressure to an inside surface of leaflet 430 (similar to pressure 260b in FIG. 2B). Control element 440 is coupled to frame 410, to anchoring junction 424, and to movable junction 422a. 422b, 422c. In some cases, control element 440 is coupled to an inner cell (not shown in FIGs. 4A-4C, but as shown in FIG. 1B), and anchoring junction 424 is coupled to the inner cell and the frame 410. In such cases, the inner cell is coupled to the movable junction 422a, 422b, 422c and the movement of the movable junction 422a, 422b, 422c causes the inner cell to move (or bend or deform, e.g., as shown in FIG. 1B), thereby causing the leaflet 430 to move (or bend or deform) as shown in FIGs. 4A-4C.

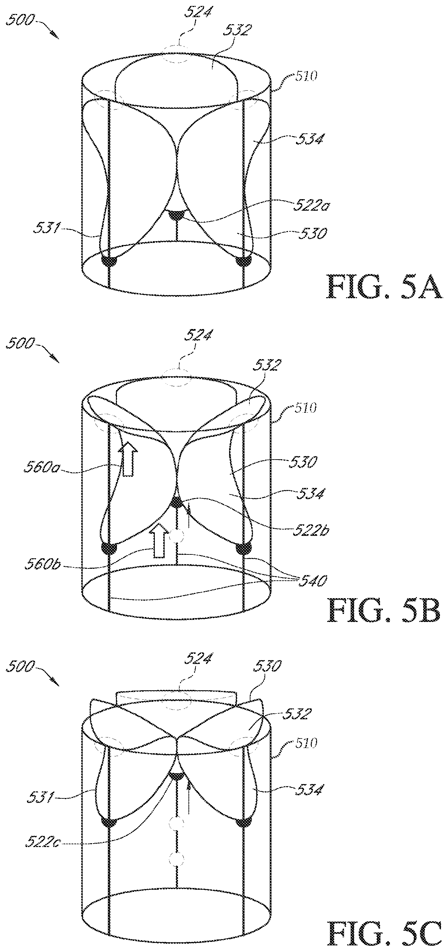

FIGs. 5A-5C show schematics of examples of portions of a flow restrictor 500. Flow restrictor 500 in FIGs. 5A-5C is similar to, and has similar components as, flow restrictor 400 in FIGs. 4A-4C. Flow restrictor 500 in FIGs. 5A-5C includes three leaflets 530, each of which is similar to leaflet 430 in FIGs. 4A-4C, and each of which are coupled to frame 510 at different locations. Leaflets 530 are shown such that outside surfaces 532, and inside surfaces 534, as well as the inflow edge 531 of the leaflets 530 are visible. In some cases, leaflets 530 can be coupled together, while in other cases, leaflets 530 can be separate and actuated separately (but may be moved together).

In some cases, leaflets 530 are coupled together (or are made from one piece of flexible material), and separate control elements 540 are used to move movable junctions 522a, 522b, 522c. However, the control elements 540 can be moved together to control the size of the lumen formed (at least in part) by leaflets 530. Some examples of leaflet shapes that can be formed from one piece of flexible material are shown in FIGs. 6B-6C. The examples in FIGs. 6B-6C show a leaflet 602 or leaflet 604 with a scalloped edge with four movable junctions 622 (e.g., that would be actively actuated by four control elements). FIGs. 6A and 6D show examples of leaflet shapes that can be separate (or can be coupled together in some cases).

The shape of the inflow edge 631 in FIGs. 6A-6C is scalloped and is relatively wide near movable junctions 622. In contrast, the shape of the inflow edge 631 in FIG. 6D is relatively narrow near the movable junction 622, which could cause more of the blood flow to apply pressure to the inside of leaflet 606 (e.g., as shown in pressure 260b applying pressure to inside 234 of leaflet 230 in FIG. 2B). In some cases, leaflet 606 in FIG. 6D can be coupled to other similar shaped leaflets to form a single leaflet with the inflow edge 631 forming multiple narrow regions coupled to multiple movable junctions (similar to how leaflet 600 in FIG. 6A can be coupled with other similar leaflets to form a single leaflet 604 in FIG. 6C).

The outflow edge 635 of the leaflets can also have different shapes in different examples. For example, leaflets 600, 604, 606 in FIGs. 6A, 6C and 6D have a curved outflow edge 635, while leaflet 602 in FIG. 6B has a straight outflow edge 635. In some cases, the outflow edge 635 can be coupled to the flow restrictor (e.g., coupled to a frame and/or an inner cell) at a point or an area 624a of the outflow edge 635, or along a substantial part (or the whole edge) 624b of the outflow edge 635.

In some cases, the leaflets of the flow restrictors described herein (e.g., in FIGs. 1A-6D) can be coupled together using a physical line (or wire). For example, the inflow edge of the leaflets (e.g., a scalloped edge, in some cases) can be formed from a physical line (e.g., a conductive wire, or shape memory material). The physical line (or wire) can couple the leaflets together such that they move together when actuated (e.g., actively, or passively). In some cases, the leaflets can be actively actuated by a control element, and the physical line (or wire) coupling the leaflets together can be part of or separate from the control element. In other cases, the leaflets can be passively actuated, and the physical line (or wire) coupling the leaflets together can help cause the leaflets to move together (e.g., in response to blood flow applying pressure to the leaflets).

In some cases, the flow restrictors described herein (e.g., in FIGs. 1A-6D) include leaflets that are separate from one another and can be actuated separately or together. In other cases, the flow restrictors described herein (e.g., in FIGs. 1A-6D) include leaflets that are coupled together, or include a single leaflet (e.g., with a scalloped edge, for example, as shown in FIGs. 6B-6C) and the coupled leaflets or single leaflet can be actuated together (e.g., by coupling an inflow edge of the leaflet(s) to one or more movable junctions).

In some cases, the flow restrictors described herein (e.g., in FIGs. 1A-6D) include one or more movable junctions that are actively actuated. In cases with more than one movable junction, there can be one active mechanism that causes all of the movable junctions to move, or there can be separate active mechanisms that cause each of the movable junctions to move independently. For example, the active mechanism to move each movable junction can include a control member including a shape memory material (e.g., nitinol) coupled to each movable junction. In some cases, a single signal (e.g., electrical current) from an electrical source can be split into multiple signals and coupled to each control mechanism such that the signal from the electrical source actuates the movable junctions together. In other cases, separate signals (e.g., electrical currents) from one or more electrical sources can be coupled to the control mechanisms such that each movable junction can be actuated separately.

In some cases, the flow restrictors described herein (e.g., in FIGs. 1A-6D) include inner cells that are coupled to a frame at an anchoring junction. In some cases, the frame can be approximately cylindrical in shape (e.g., as shown in FIGs. 4A-5C) or take on other shapes such as a lattice shape (e.g., as shown in FIG. 1A). In some cases, a change in blood pressure can cause a shape (e.g., a diameter) of the frame to change, for example due to a change in pressure in a highly compliant blood vessel. In some cases, the movable junctions (and inner cells, in some cases) are coupled to control elements that are coupled to the frame such that the change in the shape of the frame can cause the control elements to move the movable junctions, thereby causing the leaflets (and inner cells, in some cases) to bend (or deform), thereby causing the lumen to change size and the blood flow through the flow restrictor to be restricted (or modulated) in response to the change in blood pressure. For example, the control elements can be wires or rods with fixed lengths that pull and/or push the movable junctions in response to the change in shape of the frame.

In some cases, a combination of active and passive actuation can cause leaflets of flow restrictors described herein to move. For example, an active mechanism can be used to apply a first force (e.g., using a shape memory material, or other mechanism) that causes one or more movable junctions of a flow restrictor to move, and additionally, a passive mechanism can apply a second force to leaflets of the flow restrictor to further cause the one or more movable junctions of a flow restrictor to move. In such cases, the first force and the second force can be similar in magnitude, causing the first and second forces to play similar roles in moving the one or more movable junctions. In other cases, the first force can have a larger magnitude than the second force, causing the first force to play a larger role in moving the one or more movable junctions. In still other cases, the second force can have a larger magnitude than the first force, causing the second force to play a larger role in moving the one or more movable junctions.

Any of the flow restrictors described herein (e.g., in FIGs. 1A-6D) can be configured to be compatible with the IVC, the superior vena cava (SVC), or another blood vessel (e.g., a vein upstream from a renal vein). For example, any of the flow restrictors described herein (e.g., in FIGs. 1A-6D) can have frames that are sized for a certain blood vessel. Any of the flow restrictors described herein (e.g., in FIGs. 1A-6D) can be configured to operate in blood pressure ranges suitable for a certain blood vessel. For example, in the IVC normal (or low) blood pressure is from about 1 mmHg to about 15 mmHg, elevated blood pressure is from about 10 mmHg to about 25 mmHg, and exertion-related blood pressure is greater than about 25 mmHg or greater than about 30 mmHg. However, in other blood vessels, the normal, elevated and exertion-related blood pressure ranges can be different, and any of the flow restrictors described herein (e.g., in FIGs. 1A-6D) can be configured to operate in different pressure ranges. For example, the flexibility of a leaflet, or the spring constant of a spring can be chosen to actuate a component (e.g., a leaflet, flap, or inner valve) in response to changes in blood pressures within different ranges. Moreover, any of the flow restrictors described herein (e.g., in FIGs. 1A-6D) can be configured to have one mode of operation, to be bimodal, or to have more than two modes of operation. For example, in addition to a bimodal configuration by which the bimodal flow restrictors described herein can adapt blood flow restriction below (e.g., less than about 25 mmHg) and above (e.g., greater than about 30 mmHg) specific pressure values or ranges, the components of the flow restrictors (e.g., flaps, wires, frame, inner valve, etc.) can be designed to provide desired restricted or unrestricted profiles at desired pressure ranges, which can differ between more than two or three pressure ranges of a bimodal configuration.

### METHODS

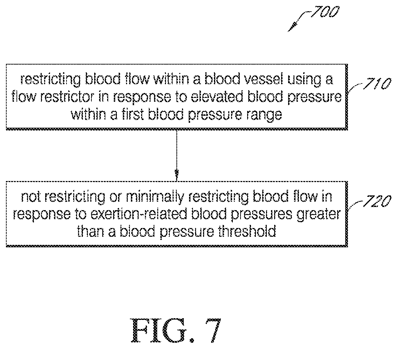

FIGs. 7-8 show examples of methods related to restricting blood flow within a blood vessel, and methods of treatment using flow modulating devices that are flow restrictors for blood vessels. The methods related to flow restrictors for blood vessels described herein can be used to occlude, partially occlude, and/or otherwise manage or regulate blood flow to or through a portion of a blood vessel (e.g., the IVC, or a blood vessel upstream from a renal vein), for example, to reduce blood pressure in blood vessels and/or organs (e.g., kidneys) downstream from the flow restrictor. The methods related to flow restrictors described herein can additionally, or alternatively, be used for any suitable applications, clinical or otherwise. For example, the methods related to flow restrictors described herein can be used for any suitable application requiring restriction of blood flow within a blood vessel, for example, to increase blood flow through an organ (e.g., the kidneys), to modulate pressure in the right atrium of the heart, and/or to reduce the accumulation of blood in the venous system.

In some examples, a method of restricting blood flow within a blood vessel includes restricting blood flow within a blood vessel using a flow restrictor described herein (e.g., in FIGs. 1A-6D), in response to elevated blood pressure.

FIG. 7 shows a flowchart of an example method 700 of restricting blood flow within a blood vessel including the following blocks. At block 710, blood flow is restricted within a blood vessel using a flow restrictor described herein (e.g., in FIGs. 1A-6D), in response to elevated blood pressure within a first blood pressure range (e.g., from about 10 mmHg to about 25 mmHg, or from about 15 mmHg to about 25 mmHg). For example, excessive blood volume in the blood vessel, or volume overload, can lead to such elevated blood pressure conditions. At block 720, blood flow is not restricted or is minimally restricted (permitting a larger amount of blood flow) through the blood vessel in response to exertion-related blood pressures greater than a blood pressure threshold (e.g., greater than about 25 mmHg, or greater than about 30 mmHg).

In some examples, a method of treatment for a subject with congestive heart failure (CHF) and/or chronic kidney disease (CKD) includes restricting blood flow within a blood vessel using a flow restrictor described herein (e.g., in FIGs. 1A-6D), in response to elevated blood pressure. In some examples, method 700 can be a method of treatment for a subject with congestive heart failure (CHF) and/or chronic kidney disease (CKD), including, at block 710, restricting blood flow within a blood vessel using a flow restrictor described herein (e.g., in FIGs. 1A-6D), in response to elevated blood pressure within a first blood pressure range (e.g., from about 10 mmHg to about 25 mmHg, or from about 15 mmHg to about 25 mmHg), and, at block 720, blood flow is not restricted or is minimally restricted (permitting a larger amount of blood flow) through the blood vessel in response to exertion-related blood pressures greater than a blood pressure threshold (e.g., greater than about 25 mmHg, or greater than about 30 mmHg).

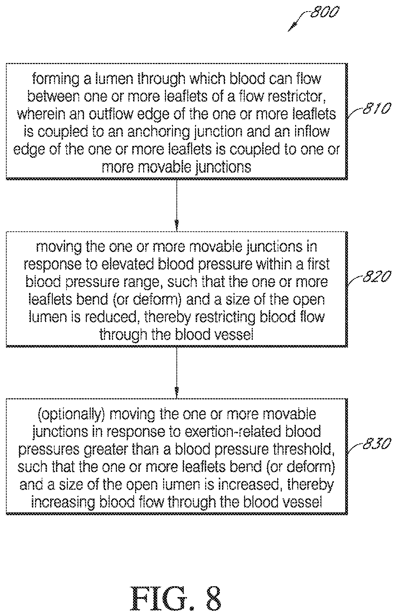

FIG. 8 shows a flowchart of an example method 800 of restricting blood flow within a blood vessel including the following blocks. At block 810, a lumen is formed through which blood can flow between one or more leaflets of a flow restrictor, wherein an outflow edge of the one or more leaflets is coupled to an anchoring junction and an inflow edge of the one or more leaflets is coupled to one or more movable junctions (e.g., as shown in the flow restrictors in FIGs. 1A-6D). At block 820, the one or more movable junctions move in response to elevated blood pressure within a first blood pressure range (e.g., from about 10 mmHg to about 25 mmHg, or from about 15 mmHg to about 25 mmHg), such that the one or more leaflets bend (or deform) and a size of the open lumen is reduced, thereby restricting blood flow though the blood vessel. For example, excessive blood volume in the blood vessel, or volume overload, can lead to such elevated blood pressure conditions. At optional block 830, the one or more movable junctions move in response to exertion-related blood pressures greater than a blood pressure threshold (e.g., about 25 mmHg or about 8 mmHg), such that the one or more leaflets bend (or deform) and a size of the open lumen is increased, thereby increasing blood flow though the blood vessel.

The one or more movable junctions and one or more leaflets (or flaps) can move at blocks 820 and 830 as described with respect to the flow restrictors described herein, for example those in FIGs. 1A-6D. For example, the one or more leaflets can prolapse at optional block 830, such that a size of the lumen is increased. In some cases, the flow restrictors of method 800 can include one or more inner cells, with each inner cell coupled to an anchoring junction and coupled to a movable junction (e.g., as shown in FIG. 1B). In such cases, the inner cell can move (or bend, or deform) as the movable junction moves (e.g., as shown in FIG. 1B). The leaflet(s) can be coupled to the inner cell(s), and therefore move with the inner cell(s) and with the movable junction(s).

Single mode flow restrictors may only be able to perform blocks 810 and 820, while bimodal flow restrictors can perform blocks 810, 820 and 830. In some cases, the one or more movable junctions can be actively moved (or actuated) at block 820 and optional block 830 in response to a sensor detecting a change in blood pressure. In other cases, the one or more movable junctions can be passively moved (or actuated) at block 820 and optional block 830 in response to changes in blood pressure applying pressure to one or more surfaces of the one or more leaflets. In still other cases, the flow restrictor of method 800 can use a combination of active and passive actuation (or movement) mechanisms.

### EXAMPLE IMPLANTATION OF FLOW MODULATING DEVICES

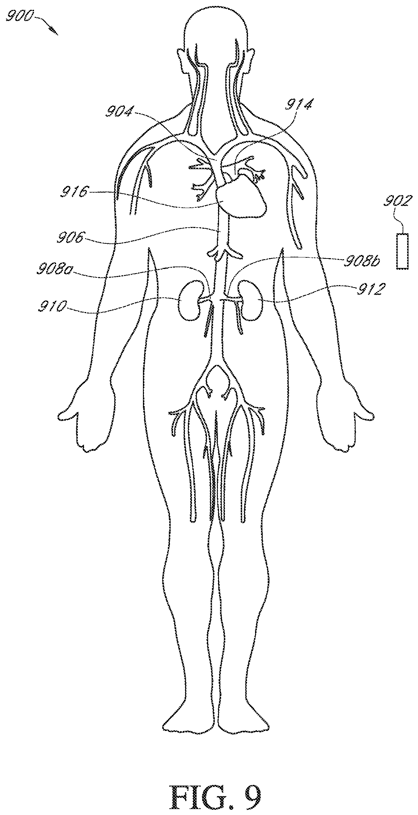

FIG. 9 illustrates a schematic representation of portions of a subject 900. The flow modulating devices described herein (represented in FIG. 9 by device 902) may be introduced (e.g., implanted) in vasculature of the body. In general, the device 902 may represent any of the flow modulating devices described herein (e.g., flow restrictors described with respect to those shown in FIGs. 1A-6D) and may include the same or similar functionality and/or structures. In some examples, the device 902 may be implanted in or near to a portion of the Superior Vena Cava (SVC) 904. In some examples, the device 902 may be implanted in or near to a portion of the Inferior Vena Cava (IVC) 906. The subject 900 is illustrated with a representation of a portion of the vasculature system to generally illustrate the SVC 904 and the IVC 906 within the subject 900. However, it is to be understood that no dimensions or relative sizes of components may be inferred from the relative sizes and dimensions of elements in the figures.

The subject 900 includes a number of vessels and organs that may circulate blood throughout the body. For example, renal veins 908a and 908b drain blood from respective right kidney 910 and left kidney 912. Renal veins 908a and 908b connect to the IVC 906. Blood from the aorta 914 flows to the IVC 906. Blood travels from the aorta 914 to the abdominal organs including the stomach (not shown), liver (not shown), spleen (not shown), pancreas (not shown), large intestines (not shown), and small intestine (not shown). Following processing of the blood by the liver, blood collects in the central vein. Blood from these central veins converges in the hepatic veins (not shown) which exit the liver and empty into the IVC 906 to be distributed to the rest of the body.

Portions of the above-recited blood circulating vessels and/or organs may be involved in splanchnic venous circulation that includes blood flow originating from the celiac, superior mesenteric, and inferior mesenteric arteries to the abdominal organs. The splanchnic venous circulation may act as a blood reservoir that can support the need for increased stressed blood volume during periods of elevated sympathetic tone, such as during exertion, to support increased cardiac output and vasodilation of peripheral vessels supporting active muscles.

However, heart failure patients can have multiple comorbidities that prevent the use of this additional blood reservoir. Example comorbidities can include chronic kidney disease, chronotropic incompetence, inability to increase stroke volume, and/or peripheral microvascular dysfunction. This can lead to venous congestion and/or abrupt rises in central venous pressure, pulmonary artery pressure, and/or pulmonary capillary wedge pressure. To alleviate such pressures, the blood reserves within the blood reservoir described above can be used to support the need for increased stressed blood volume during periods of elevated sympathetic tone. The flow modulating devices described herein may be used to ensure that such blood reserves within the blood reservoir can be utilized.

For example, because blood flow from the splanchnic venous circulation is directed through hepatic veins and into the IVC 906, devices (as described herein) may be placed into the IVC 906 to limit blood flow to allow the reservoir to expand with increased blood volume. Similarly, devices (as described herein) may be placed into the SVC 908 to limit blood flow to allow the reservoir to expand with increased blood volume. Furthermore, the flow modulating devices described herein may be placed in either the IVC 906 and/or SVC 908 to alleviate pressure in the right side of the atrium of the heart 916.

In some examples, the flow modulating device 902 (representing the devices described herein) may be used as a method of treatment to treat any combination of heart failure, chronic kidney disease, chronotropic incompetence, inability to increase stroke volume, and/or peripheral microvascular dysfunction. In addition, the flow modulating device 902 may be used as a method of treatment to regulate pressure in the right atrium of the heart. Further, the flow modulating device 902 may be used as a method of treatment to improve function of the kidneys in patients having reduced kidney function due to pressure in the venous system.

In some embodiments, a method of treatment can include using one or more methods and/or devices described herein to facilitate management of blood flow through and/or into one or more blood vessels and/or chambers of a heart. There is a need for new and useful method of treatment using flow restrictors for blood vessels. In particular, there is a need for methods of treatment using the systems, devices, and methods described herein, the methods including modulating and/or balancing of blood flow through a blood vessel, for example, to occlude, partially occlude, and/or otherwise manage or regulate blood flow to or through a portion of a blood vessel.

In some embodiments, a method of treatment for restricting blood flow within a blood vessel includes using a flow restrictor for a blood vessel. The flow restrictor comprises: a frame positionable within a blood vessel; an inner cell; a leaflet; and a lumen through the flow restrictor, wherein the lumen is defined at least in part by the leaflet. The frame can comprise an outer cell comprising an inflow end and an outflow end. The inner cell can comprise an anchoring junction coupled to an outflow end of the outer cell, and a movable junction. The leaflet can comprise an inflow edge and an outflow edge. The inflow edge is coupled to the movable junction of the inner cell, and the outflow edge is coupled to the anchoring junction. The movable junction of the inner cell and the inflow edge of the leaflet can be configured to move axially in response to a first increase in a blood pressure within the blood vessel, and in response to the blood pressure within the blood vessel being within a first blood pressure range. The leaflet can be curved and can be configured to bend in response to the movable junction moving axially. The movable junction and the leaflet can be configured such that the lumen changes size when the movable junction moves.

As used herein, the term "active" with respect to blood flow management may represent operations carried out by the devices described herein using power or controller induced movement. For example, actively moving a portion of the devices described herein may include the use of battery power, wall outlet power, magnetic field induction, electromagnetic field induction, magnetic polarization, a piston-based system, a valve-based system (e.g., with a manifold), hydraulics, pneumatics, optical actuators, thermal actuators, and/or other actuator using electrical or inductive power.

In some implementations, an active control mechanism may include a microcontroller and/or a power source implanted with or integrated with the flow management device. Alternatively, or additionally, an active control mechanism can include a microcontroller and/or a power source in a remote control device, external to the body, or in an implanted remote device (e.g., subcutaneously, intravascularly, etc.), for example. The remote control device may be in wireless communication with the implanted device or connected to the implanted device through one or more leads.

In any of the embodiments described herein, an active mechanism may include a pump fluidly connected to a reservoir; a chamber having a first portion and a second portion; a manifold fluidly connected to the pump, the reservoir, and the chamber; and a piston coupled to a control element of a flow modulating device. The manifold may include at least one port that fluidly connects the reservoir to the first portion of the chamber. The piston can move between a restricted blood flow position and an unrestricted blood flow position within the chamber, any position therebetween for intermediate blood flow restriction positions. For example, the piston may move to the restricted blood flow position when a fluid is flowed from the reservoir, charged by a pump, through the manifold into the first portion of the chamber. The piston can return to the unrestricted blood flow position when the fluid is evacuated from the first portion of the chamber. In some embodiments, the manifold is fluidly connected to a second portion of the chamber through a second port. In such embodiments, the piston can move to the unrestricted blood flow position when the fluid enters the second port from the reservoir through the manifold, thereby causing the valve of the flow modulating device to move to the unrestricted blood flow state. In some embodiments, the fluid is evacuated from the second portion of the chamber through the second port when the piston is in the restricted blood flow position. In some implementations, the at least one port further fluidly connects the first portion of the chamber to the pump through the manifold. For example, the at least first port is fluidly connected to the pump through the manifold to evacuate the fluid from the first portion of the chamber thereby moving the piston to the unrestricted blood flow position. In some examples, the piston is a spring-based piston. For example, the spring-based piston can automatically return to the unrestricted blood flow position when the fluid is evacuated from the first portion of the chamber.

In any of the embodiments described herein, an active mechanism may include a linear actuator coupled to a control element of the flow management device. The linear actuator tensions the control element to position the valve of the flow management device in the restricted blood flow state. Alternatively, the linear actuator releases tension in the control element to position the valve in the unrestricted blood flow state. The tensioning and releasing of tension on the control element may be based on a predefined set of parameters or based on a sensed attribute of the blood vessel in which the flow management device is implanted. For example, the sensed attribute may be sensed by a sensor. The sensor may be coupled to the flow management device, a remote control device, or otherwise in wireless or electrical communication with a flow management system. The sensor can be a strain gauge, a piezoelectric sensor, a capacitance sensor, or a vacuum pressure sensor, such that the sensor senses a pressure in the blood vessel.

In any of the embodiments described herein, the linear actuator is an electromechanical linear actuator having a first magnet that, when caused to rotate by another magnet or actuator, causes a nut to rotate on a lead screw, the nut being coupled to the control element. A second magnet in a control device may cause rotation of the first magnet, for example by changing its magnetic field pole direction. In some embodiments, a repeater magnet (with or without its own power source) is positioned between the first magnet and the second magnet, for example in cases where the first magnet is beyond a threshold distance from the second magnet.

In any of the embodiments described herein, the linear actuator is a pneumatic linear actuator having a piston coupled to the control element. Injecting compressed gas moves the piston to tension the control element to move the valve into a restricted blood flow state and venting the compressed gas releases tension in the control element to move the valve to an unrestricted blood flow state.

In any of the embodiments described herein, the linear actuator is a hydraulic linear actuator having a piston coupled to the control element. Injecting liquid moves the piston to tension the control element to move the valve into a restricted blood flow state and venting the liquid releases tension in the control element to move the valve to an unrestricted blood flow state.

In any of the embodiments described herein, the linear actuator is a thermal linear actuator having a piston coupled to the control element. For example, increasing a temperature of a thermal sensitive fluid (e.g., via a heat source, changes in body temperature, etc.) causes the piston to compress the fluid to tension the control element to move the valve into the restricted blood flow state. Alternatively, decreasing the temperature of the thermal sensitive fluid causes the piston to decompress the fluid to release tension in the control element to move the valve to the unrestricted blood flow state.

In any of the embodiments described herein, the linear actuator comprises a shape-memory material (e.g., nickel titanium alloys, or nitinol). The shape memory material can be coupled to the control element, or may form all, or a part, of the control element. An electrical current applied to the shape memory material can cause it to change size, length or shape, thereby causing the control element to move or be actuated (e.g., in response to a signal from a sensor).

As used herein, the term "passive" with respect to blood flow management may represent operations carried out by the devices described herein using passively induced movement. For example, passively moving a portion of the devices described herein may include the use of manual pull wires (e.g., sutures, actuation wires/cords, etc.), anatomy responses (e.g., changes in vessel inner diameter, intra-vessel pressure, etc.), blood movement, or the like.

In some examples described herein, a passive mechanism can include a control element, such as a rigid push or pull member (e.g., a rod or rigid wire), a flexible tension member, a flexible wire, a suture, a string, a cable, or the like. One or more control elements can be coupled to a body to be moved (or actuated), such as one or more leaflets, flaps, valves, or valve portions (e.g., used to restrict blood flow through a blood vessel) and to a frame (or other component). For example, the control element can be coupled to a frame that moves in response to an external force (e.g., an increase in blood pressure). In some examples, the control element is coupled to a helical tube that expands and contracts with a blood vessel in which it is positioned, and the movement of the helical tube moves the control element. In some examples, a control member can be used to actuate the valve in response to the movement of the frame (or helical tube) due to a change in blood pressure within a blood vessel.

In some examples described herein, a passive mechanism can include a spring, or elastic member (such as a flexible commissure). One or more springs or elastic members can be coupled to a body to be moved (or actuated), such as one or more leaflets, flaps, valves, or valve portions (e.g., used to restrict blood flow through a blood vessel) and to a frame. For example, a spring can be used to bias the leaflet, flap, or valve in an initial position (e.g., under relatively lower blood pressure conditions). An externally applied force can cause the leaflet, flap, or valve to move and extend the spring (e.g., under relatively higher blood pressure conditions, for example wherein increased blood flow applies pressure against the leaflet, flap, or valve). In the absence of the applied force, the spring can recompress, thereby bringing the leaflet, flap, or valve back to its initial position.

In some examples described herein, a passive mechanism can include more than one mode of operation. For example, a body (or member or component) of a blood flow regulator (or restrictor) described herein can move in a particular way in response to a first externally applied force, and then the body (or member or component) of a blood flow regulator (or restrictor) described herein can move in a different way in response to a second externally applied force. In some cases, the first externally applied force and the second externally applied force are applied from the same external force (e.g., blood pressure), with different quantitative ranges. For example, a leaflet, flap, or valve (e.g., used to restrict blood flow through a blood vessel) can move in a first mode in response to an increase in blood pressure within a first blood pressure range, and move in a second mode in response to an increase in blood pressure within a second blood pressure range that is different (e.g., higher) than the first blood pressure range. The first mode can be that an end of the leaflets, flaps, or valve portions moves towards one another to further restrict blood flow within the blood vessel, and the second mode can be that the leaflets, flaps, or valve portions prolapse, thereby moving away from one another to increase the blood flow within the blood vessel.

In some examples described herein, a flow modulating device (e.g., a flow restrictor) described herein can include more than one passive mechanism. For example, a flow modulating device can include a first passive mechanism that can move in a first mode in response to an increase in blood pressure within a first blood pressure range, and a second passive mechanism that can move in a second mode in response to an increase in blood pressure within a second blood pressure range that is different (e.g., higher or maximum) than the first blood pressure range. The first passive mechanism can include leaflets, flaps, or valve portions that move towards one another to further restrict blood flow within the blood vessel, and the second passive mechanism can include an inner valve that can move (e.g., axially) to open additional channels through the flow regulator device to increase the blood flow. In some cases, the inner valve can be biased using a passive element such as one or more springs or spring like elements, such that it will return to its initial position after the blood pressure decreases (e.g., back into a first blood pressure range).

Any of the implantable or flow modulating devices described herein may be coated with a polymer (e.g., silicones, poly(urethanes), poly(acrylates), or copolymers such as poly(ethylene vinyl acetate), a drug (e.g., heparin, pro-endothelialization drugs, anti-thrombogenic drug, etc.), a textile (e.g., woven, knitted, nonwoven, or braided), tissue (e.g., bovine pericardium, equine pericardium, porcine vena cava, etc.), or a combination thereof. Woven and knitted fabrics may be made from poly (ethylene terephthalate), while the nonwoven fabrics may be made from expanded poly(tetrafluoroethylene). Some textiles may also or alternatively include silk or silk-based materials.

Further, any of the pull wires, sutures, or actuation wires described herein may include silk, silk-based materials, nylon, synthetic polymer materials (e.g., silicone, polydioxanone, polyglycolic acid, polyglyconate, polylactic acid, etc.), natural materials (e.g., purified catgut, collagen, sheep intestines, cow intestines, etc.), metal (e.g., Nitinol, palladium, gold and their alloys, etc.), or a combination thereof.

The flow modulating devices described herein may be part of (or installed within) a stent. The stent may represent a frame or outer frame that provides a support structure for the flow modulating devices when the stent is implanted into a blood vessel. The frame/outer frame may be a self-expanding frame or a balloon-expandable frame. In general, any type of stent may be used with the flow modulating devices. Example stents may include, but are not limited to, bare metal stents, coated stents, drug-eluting stents, biodegradable stents, balloon expandable stents, and self-expandable stents.

The stents described herein may be configured to house all or a portion of the flow modulating devices described herein. Such stents may include an assembly with strut members interconnected by joints that form a series of linked mechanisms that result in a hollow tube-shaped element. The stents may be positioned and/or repositioned within a blood vessel to introduce or remove flow modulating devices or device members including, but not limited, to valving, control elements, balloons, flexible members, rigid members, adjustment mechanisms, sensors, coils, wires, and/or magnets. One or more of such device members may be actuated to modify stent shape (or device member shape) for purposes of modifying a flow of fluid through the vessel associated with the implanted stent. Moreover, the stents described herein may partially or fully surround a flow modulating device. For example, a stent or stent portion may surround a portion of a flow modulating device to ensure the device remains in a specified position in a blood vessel. In some examples, the stent surrounds the flow modulating device entirely. In some examples, the stent surrounds the flow modulating device and further continues beyond one or both ends of the device.

The stents described herein may include an outer frame. The outer frame may have a form and structure that varies. For example, the strut members and/or articulated joints may form a mesh-like structure. The strut members may be interconnected in such a way as to form a shaped pattern of cells. For example, any number of strut members may form a ring of the stent such that the strut members are connected by any number of crowns. Any number of rings may form a body of the stent, and the rings may be connected by any number of bridges. Example cell shapes may include, but are not limited to diamond, square, rectangle, triangle, oval, ganglion, or any combination thereof. In some examples, the cells may be evenly shaped and distributed from a first end of the stent to a second end of the stent. In some examples, the cells may include a number of strut members interconnected in such a way that when the stent expands radially, one or more of the cells become longitudinally shorter. Similarly, when the stent constricts radially, one or more of the cells become longitudinally longer.

Constricting portions of the stents described herein may result in an outer frame woven tighter than other portions of the stent that are not constricted. The constriction may push against one or more portions of the flow modulating devices described herein to narrow a pathway through the frame or outer frame and/or to trigger the flow modulating device to begin or end constriction. Similarly, expanding portions of the stents described herein may result in an outer frame woven looser than other portions of the stent that are not expanded. The expansion may release one or more portions of the flow modulating devices described herein to widen a pathway through the frame or outer frame and/or to trigger the flow modulating device to begin or end constriction.

The flow modulating devices described herein may be introduced to a vessel or tissue site using a delivery system. For example, such delivery systems may be used to position catheter tips and/or catheters in various portions of a target vasculature. A delivery system may include a delivery catheter having a pusherwire or the like disposed therein. The pusherwire may be configured to deploy any of the devices described herein, for example by urging the device out of a distal end of the catheter and either actively expanding the device or allowing the device to passively expand once it is no longer constrained by a lumen of the catheter. Any of the devices described herein may be crimped or otherwise compressed such that a cross-sectional area of the device is sized and/or shaped to be delivered through a lumen of a catheter. In some examples, the crimped or compressed device may be transferred to the delivery system using a transfer sheath, or the like. A delivery system can access the vasculature through an access site, such as a radial artery, brachial artery, internal jugular vein, common femoral vein, subclavian veins, or the like.

For example, in a coronary procedure, a catheter tip and/or catheter may be configured to pass from the right atrium into the coronary sinus. For access to the venous circulation, for example, a catheter tip and/or catheter may be configured to pass from the radial artery into the superior vena cava. Further, for central venous access, a catheter tip and/or catheter may be configured to pass from the femoral vein into the inferior vena cava.

In some examples, the delivery system may include a trocar or other suitable delivery device may be used for implanting devices subcutaneously, for example control devices for controlling activation of any of the flow modulating devices described herein. As described elsewhere herein, various control systems may include an implanted remote device that is configured to transmit control signals to a flow modulating device disposed in the vasculature. The control signals may include signals transmitted wirelessly, through a wired connection (e.g., leads), or via magnetic field induction, electromagnetic field induction, or magnetic polarization.

However, it will be understood that the delivery system can refer or generally apply to positioning of catheter tips and/or catheters from a first body chamber or lumen into a second body chamber or lumen, where the catheter tips and/or catheters may be bent when positioned from the first body chamber or lumen into the second body chamber or lumen. A body chamber or lumen can refer to any one of a number of fluid channels, blood vessels (e.g., superior vena cava, inferior vena cava, renal artery, renal vein, etc.), and/or organ chambers (e.g., heart chambers). Additionally, reference herein to "catheters," "tubes," "sheaths," "steerable sheaths," and/or "steerable catheters" can refer or apply generally to any type of elongate tubular delivery device including an inner lumen configured to slidably receive instrumentation, such as for positioning within an atrium, coronary sinus, superior vena cava, or inferior vena cava, including for example delivery catheters, cannulas, and/or trocars. It will be understood that other types of medical implant devices and/or procedures can be delivered to the coronary sinus, superior vena cava, inferior vena cava, etc. using a delivery system as described herein, including for example ablation procedures, drug delivery, and/or placement of actuator leads.

Described herein are various example medical implants and/or delivery methods. Some examples described herein may be used in combination and/or may be used independently.

The spatially relative terms "outer," "inner," "upper," "lower," "below," "above," "vertical," "horizontal," and similar terms, may be used herein for ease of description to describe the relations between one element or component and another element or component as illustrated in the drawings. It should be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

The systems and methods of the embodiments and variations described herein can be embodied and/or implemented at least in part as a machine configured to receive a computer-readable medium storing computer-readable instructions. The instructions may be executed by computer-executable components integrated or in communication with the system and one or more portions of the processor on or in communication with any device described herein (e.g., a flow restrictor of FIGs. 1A-6D) and/or computing device. For example, the flow restrictors described herein may be controllable by a control device such that the control device includes computer-executable components configured to execute the instructions. The flow restrictors may be in wireless or wired (e.g., via a lead) communication with the control device. The computer-readable medium can be stored on any suitable computer-readable media such as RAMs, ROMs, flash memory, EEPROMs, optical devices (e.g., CD or DVD), hard drives, floppy drives, or any suitable device. The computer-executable component is preferably a general or application-specific processor, but any suitable dedicated hardware or hardware/firmware combination can alternatively or additionally execute the instructions.

As used in the description and claims, the singular form "a", "an" and "the" include both singular and plural references unless the context clearly dictates otherwise. For example, the term "flap" or "leaflet" may include, and is contemplated to include, a plurality of flaps or leaflets. At times, the claims and disclosure may include terms such as "a plurality," "one or more," or "at least one;" however, the absence of such terms is not intended to mean, and should not be interpreted to mean, that a plurality is not conceived.

The term "about" or "approximately," when used before a numerical designation or range (e.g., to define a length or pressure), indicates approximations which may vary by ( + ) or ( - ) 5%, 1% or 0.1%. All numerical ranges provided herein are inclusive of the stated start and end numbers. The term "substantially" indicates mostly (i.e., greater than 50%) or essentially all of a device, substance, or composition.

As used herein, the term "comprising" or "comprises" is intended to mean that the devices, systems, and methods include the recited elements, and may additionally include any other elements. "Consisting essentially of" shall mean that the devices, systems, and methods include the recited elements and exclude other elements of essential significance to the combination for the stated purpose. Thus, a system or method consisting essentially of the elements as defined herein would not exclude other materials, features, or steps that do not materially affect the basic and novel characteristic(s) of the claimed disclosure. "Consisting of" shall mean that the devices, systems, and methods include the recited elements and exclude anything more than a trivial or inconsequential element or step. Embodiments defined by each of these transitional terms are within the scope of this disclosure.

The examples and illustrations included herein show, by way of illustration and not of limitation, specific embodiments in which the subject matter may be practiced. Other embodiments may be utilized and derived therefrom, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is in fact disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. The invention relates to a flow restrictor for a blood vessel as defined in claim 1, Embodiments of the invention are recited in the dependent claims.

## Claims

1. A flow restrictor (100) for a blood vessel, comprising:
a frame (110) positionable within a blood vessel, wherein the frame comprises an outer cell comprising an inflow end and an outflow end;
an inner cell (120) comprising an anchoring junction (124) coupled to the outflow end of the outer cell, and wherein the inner cell comprises a movable junction (122);
a leaflet (130) comprising an inflow edge and an outflow edge, wherein the inflow edge of the leaflet is coupled to the movable junction of the inner cell, and wherein the outflow edge of the leaflet is coupled to the anchoring junction; and
a lumen (350a) through the flow restrictor, wherein the lumen is defined at least in part by the leaflet,
wherein the movable junction of the inner cell and the inflow edge of the leaflet are configured to move axially in response to a first increase in a blood pressure within the blood vessel, and in response to the blood pressure within the blood vessel being within a first blood pressure range,
wherein the leaflet is curved and is configured to bend in response to the movable junction moving axially, and
wherein the movable junction and the leaflet are configured such that the lumen changes size when the movable junction moves.

2. The flow restrictor of claim 1, further comprising a second outer cell, a second inner cell comprising a second movable junction, and a second leaflet, wherein an inflow edge of the second leaflet is coupled to the second movable junction.

3. The flow restrictor of claim 2, further comprising two or more outer cells, two or more inner cells each comprising a movable junction, and two or more leaflets, wherein an inflow edge of each of the two or more leaflets is coupled to a movable junction,
optionally wherein the two or more leaflets are coupled together and the inflow edges of the two or more leaflets form a scalloped edge.

4. The flow restrictor of any preceding claim, further comprising a conductive wire, wherein the movable junction is coupled to the conductive wire, wherein the movable junction is configured to move axially from a first position to a second position in response to a first electrical current applied to the conductive wire, wherein the second position is closer to the outflow end of the outer cell than the first position, and wherein the leaflet is configured to bend such that the lumen is smaller when the movable junction is in the second position than it is when the movable junction is in the first position,
optionally wherein the conductive wire is:
a) configured to change length or change shape in response to the first electrical current; or
b) a shape-memory alloy, or nitinol.

5. The flow restrictor of claim 4, further comprising a sensor configured to measure the blood pressure within the blood vessel, wherein the sensor is a strain gauge, a piezoelectric sensor, a capacitance sensor, or a vacuum pressure sensor, and wherein the first electrical current is applied in response to a signal from the sensor.

6. The flow restrictor of claim 4, wherein the movable junction is configured to move axially from the second position to a third position in response to a second electrical current applied to the conductive wire, wherein the third position is closer to the outflow end of the outer cell than the second position, wherein the leaflet is configured to prolapse or collapse when the movable junction is in the third position such that the lumen is larger when the movable junction is in the third position than it is when the movable junction is in the second position.

7. The flow restrictor of claim 6, wherein the second electrical current is applied in response to a second increase in blood pressure within the blood vessel, and in response to the blood pressure within the blood vessel being within a second blood pressure range, wherein a lower limit of the second blood pressure range is higher than an upper limit of the first blood pressure range.

8. The flow restrictor of claim 7, wherein the first blood pressure range is associated with a volume overload blood pressure, and the second blood pressure range is associated with an exertion-related blood pressure.

9. The flow restrictor of claim 7 or claim 8, further comprising a sensor configured to measure the blood pressure within the blood vessel, wherein the sensor is a strain gauge, a piezoelectric sensor, a capacitance sensor, or a vacuum pressure sensor, and wherein the first electrical current and the second electrical current are applied in response to signals from the sensor.

10. The flow restrictor of any preceding claim, wherein blood flow within the blood vessel can contact an outside surface (232) of the leaflet, or an inside surface (234) and the outside surface (232) of the leaflet, wherein each movable junction is configured to move axially from a first position to a second position in response to the blood pressure applying a first pressure to the outside surface or the inside surface and the outside surface of the leaflet, wherein the second position is closer to the outflow end of the outer cell than the first position, and wherein the lumen is smaller when the movable junction is in the second position than it is when the movable junction is in the first position.

11. The flow restrictor of claim 10, wherein the movable junction is configured to move axially from the second position to a third position in response to the blood pressure applying a second pressure to the outside surface or the inside surface and the outside surface of the leaflet, and in response to the blood pressure within the blood vessel being within a second blood pressure range, wherein a lower limit of the second blood pressure range is higher than an upper limit of the first blood pressure range, wherein the third position is closer to the outflow end of the outer cell than the second position, wherein the leaflet is configured to prolapse or collapse when the movable junction is in the third position, and wherein the lumen is larger when the movable junction is in the third position than it is when the movable junction is in the second position.

12. The flow restrictor of claim 11, wherein the first blood pressure range is associated with a volume overload blood pressure, and the second blood pressure range is associated with an exertion-related blood pressure,
optionally wherein the first blood pressure range is from 10 mmHg to 25 mmHg and the second blood pressure range is greater than about 25 mmHg or greater than about 30 mmHg.

13. The flow restrictor of claim 10, wherein the inner cell is configured to change length or change shape in response to the first pressure.

14. The flow restrictor of any preceding claim, wherein the blood vessel is an inferior vena cava or a vein upstream from a renal vein.

15. The flow restrictor of any preceding claim, wherein the movable junction is moved using a control element (140) or a pull wire coupled to an active control mechanism,
optionally wherein the active control mechanism comprises one or more of: a microcontroller, a battery, a wall outlet power cable, a wired or wireless communication system, a magnetic field induction system, an electromagnetic field induction system, a magnetic polarization system, a linear actuator, a sensor, a piston based system, a valve based system, hydraulics, pneumatics, optical actuators, thermal actuators, and other actuator using electrical or inductive power.

## Patentansprüche

1. Durchflussbegrenzer (100) für ein Blutgefäß, umfassend:
einen Rahmen (110), der innerhalb eines Blutgefäßes positionierbar ist, wobei der Rahmen eine Außenzelle umfasst, die ein Einströmende und ein Ausströmende umfasst;
eine Innenzelle (120), die eine Verankerungsverbindungsstelle (124) umfasst, die mit dem Ausströmende der Außenzelle gekoppelt ist, und wobei die Innenzelle eine bewegbare Verbindungsstelle (122) umfasst;
ein Segel (130), das eine Einströmkante und eine Ausströmkante umfasst, wobei die Einströmkante des Segels mit der bewegbaren Verbindungsstelle der Innenzelle gekoppelt ist, und wobei die Ausströmkante des Segels mit der Verankerungsverbindungsstelle gekoppelt ist; und
ein Lumen (350a) durch den Durchflussbegrenzer hindurch, wobei das Lumen zumindest teilweise durch das Segel definiert ist,
wobei die bewegbare Verbindungsstelle der Innenzelle und die Einströmkante des Segels dazu ausgestaltet sind, sich in Reaktion auf einen ersten Anstieg eines Blutdrucks innerhalb des Blutgefäßes und in Reaktion darauf, dass der Blutdruck innerhalb des Blutgefäßes innerhalb eines ersten Blutdruckbereichs liegt, axial zu bewegen,
wobei das Segel gekrümmt ist und dazu ausgestaltet ist, sich in Reaktion darauf, dass sich die bewegbare Verbindungsstelle axial bewegt, zu biegen, und
wobei die bewegbare Verbindungsstelle und das Segel so ausgestaltet sind, dass das Lumen die Größe ändert, wenn sich die bewegbare Verbindungsstelle bewegt.

2. Durchflussbegrenzer nach Anspruch 1, der ferner eine zweite Außenzelle, eine zweite Innenzelle, die eine zweite bewegbare Verbindungsstelle umfasst, und ein zweites Segel umfasst, wobei eine Einströmkante des zweiten Segels mit der zweiten bewegbaren Verbindungsstelle gekoppelt ist.

3. Durchflussbegrenzer nach Anspruch 2, der ferner zwei oder mehr Außenzellen, zwei oder mehr Innenzellen, die jeweils eine bewegbare Verbindungsstelle umfassen, und zwei oder mehr Segel umfasst, wobei eine Einströmkante jedes der zwei oder mehr Segel mit einer bewegbaren Verbindungsstelle gekoppelt ist,
wobei gegebenenfalls die zwei oder mehr Segel miteinander gekoppelt sind und die Einströmkanten der zwei oder mehr Segel eine rundgezackte Kante bilden.

4. Durchflussbegrenzer nach einem der vorhergehenden Ansprüche, der ferner einen leitfähigen Draht umfasst, wobei die bewegbare Verbindungsstelle mit dem leitfähigen Draht gekoppelt ist, wobei die bewegbare Verbindungsstelle dazu ausgestaltet ist, sich in Reaktion auf einen ersten elektrischen Strom, der an den leitfähigen Draht angelegt wird, axial von einer ersten Position zu einer zweiten Position zu bewegen, wobei die zweite Position näher an dem Ausströmende der Außenzelle als die erste Position ist, und wobei das Segel dazu ausgestaltet ist, sich so zu biegen, dass das Lumen kleiner ist, wenn sich die bewegbare Verbindungsstelle in der zweiten Position befindet, als es ist, wenn sich die bewegbare Verbindungsstelle in der ersten Position befindet,
wobei gegebenenfalls der leitfähige Draht wie folgt ist:
a) ausgestaltet zum Ändern der Länge oder der Formgebung in Reaktion auf den ersten elektrischen Strom; oder
b) eine Formgedächtnislegierung oder Nitinol.

5. Durchflussbegrenzer nach Anspruch 4, der ferner einen Sensor umfasst, der dazu ausgestaltet ist, den Blutdruck innerhalb des Blutgefäßes zu messen, wobei der Sensor ein Dehnungsmessstreifen, ein piezoelektrischer Sensor, ein Kapazitätssensor oder ein Vakuumdrucksensor ist, und wobei der erste elektrische Strom in Reaktion auf ein Signal von dem Sensor angelegt wird.

6. Durchflussbegrenzer nach Anspruch 4, wobei die bewegbare Verbindungsstelle dazu ausgestaltet ist, sich in Reaktion auf einen zweiten elektrischen Strom, der an den leitfähigen Draht angelegt wird, axial von der zweiten Position zu einer dritten Position zu bewegen, wobei die dritte Position näher an dem Ausströmende der Außenzelle als die zweite Position ist, wobei das Segel dazu ausgestaltet ist, zu prolabieren oder zu kollabieren, wenn sich die bewegbare Verbindungsstelle in der dritten Position befindet, so dass das Lumen größer ist, wenn sich die bewegbare Verbindungsstelle in der dritten Position befindet, als wenn sich die bewegbare Verbindungsstelle in der zweiten Position befindet.

7. Durchflussbegrenzer nach Anspruch 6, wobei der zweite elektrische Strom in Reaktion auf einen zweiten Anstieg des Blutdrucks innerhalb des Blutgefäßes und in Reaktion darauf, dass der Blutdruck innerhalb des Blutgefäßes innerhalb eines zweiten Blutdruckbereichs liegt, angelegt wird, wobei eine untere Grenze des zweiten Blutdruckbereichs höher als eine obere Grenze des ersten Blutdruckbereichs ist.

8. Durchflussbegrenzer nach Anspruch 7, wobei der erste Blutdruckbereich mit einem Volumenüberlastungsblutdruck assoziiert ist und der zweite Blutdruckbereich mit einem anstrengungsbezogenen Blutdruck assoziiert ist.

9. Durchflussbegrenzer nach Anspruch 7 oder Anspruch 8, der ferner einen Sensor umfasst, der dazu ausgestaltet ist, den Blutdruck innerhalb des Blutgefäßes zu messen, wobei der Sensor ein Dehnungsmessstreifen, ein piezoelektrischer Sensor, ein Kapazitätssensor oder ein Vakuumdrucksensor ist, und wobei der erste elektrische Strom und der zweite elektrische Strom in Reaktion auf Signale von dem Sensor angelegt werden.

10. Durchflussbegrenzer nach einem der vorhergehenden Ansprüche, wobei der Blutfluss innerhalb des Blutgefäßes eine Außenfläche (232) des Segels oder eine Innenfläche (234) und die Außenfläche (232) des Segels berühren kann, wobei jede bewegbare Verbindungsstelle dazu ausgestaltet ist, sich in Reaktion darauf, dass der Blutdruck einen ersten Druck auf die Außenfläche oder die Innenfläche und die Außenfläche des Segels ausübt, axial von einer ersten Position zu einer zweiten Position zu bewegen, wobei die zweite Position näher an dem Ausströmende der Außenzelle als die erste Position ist, und wobei das Lumen kleiner ist, wenn sich die bewegbare Verbindungsstelle in der zweiten Position befindet, als es ist, wenn sich der bewegbare Verbindungsstelle in der ersten Position befindet.

11. Durchflussbegrenzer nach Anspruch 10, wobei die bewegbare Verbindungsstelle dazu ausgestaltet ist, sich in Reaktion darauf, dass der Blutdruck einen zweiten Druck auf die Außenfläche oder die Innenfläche und die Außenfläche des Segels ausübt, und in Reaktion darauf, dass sich der Blutdruck innerhalb des Blutgefäßes innerhalb eines zweiten Blutdruckbereichs befindet, axial von der zweiten Position in eine dritte Position zu bewegen, wobei eine untere Grenze des zweiten Blutdruckbereichs höher als eine obere Grenze des ersten Blutdruckbereichs ist, wobei die dritte Position näher an dem Ausströmende der Außenzelle als die zweite Position ist, wobei das Segel dazu ausgestaltet ist, zu prolabieren oder kollabieren, wenn sich die bewegbare Verbindungsstelle in der dritten Position befindet, und wobei das Lumen größer ist, wenn sich die bewegbare Verbindungsstelle in der dritten Position befindet, als es ist, wenn sich der bewegbare Verbindungsstelle in der zweiten Position befindet.

12. Durchflussbegrenzer nach Anspruch 11, wobei der erste Blutdruckbereich mit einem Volumenüberlastungsblutdruck assoziiert ist und der zweite Blutdruckbereich mit einem anstrengungsbezogenen Blutdruck assoziiert ist,
wobei gegebenenfalls der erste Blutdruckbereich 10 mmHg bis 25 mmHg beträgt und der zweite Blutdruckbereich größer als etwa 25 mmHg oder größer als etwa 30 mmHg ist.

13. Durchflussbegrenzer nach Anspruch 10, wobei die Innenzelle dazu ausgestaltet ist, in Reaktion auf den ersten Druck die Länge zu ändern oder die Formgebung zu ändern.

14. Durchflussbegrenzer nach einem der vorhergehenden Ansprüche, wobei das Blutgefäß eine Vena cava inferior oder eine Vene stromaufwärts von einer Nierenvene ist.

15. Durchflussbegrenzer nach einem der vorhergehenden Ansprüche, wobei die bewegbare Verbindungsstelle unter Verwendung eines Steuerelements (140) oder eines Zugdrahts bewegt wird, der mit einem aktiven Steuermechanismus gekoppelt ist,
wobei gegebenenfalls der aktive Steuermechanismus eines oder mehrere von Folgendem umfasst: einen Mikrocontroller, eine Batterie, ein Steckdosenstromkabel, ein drahtgebundenes oder drahtloses Kommunikationssystem, ein Magnetfeldinduktionssystem, ein Elektromagnetfeldinduktionssystem, ein Magnetpolarisationssystem, einen Linearaktuator, einen Sensor, ein kolbenbasiertes System, ein ventilbasiertes System, Hydraulik, Pneumatik, optische Aktuatoren, thermische Aktuatoren und andere Aktuatoren mit elektrischer oder induktiver Leistung.

## Revendications

1. Limiteur de débit (100) pour un vaisseau sanguin, comprenant :
une structure (110) pouvant être positionnée à l'intérieur d'un vaisseau sanguin, dans lequel la structure comprend une cellule externe comprenant une extrémité d'entrée et une extrémité de sortie ;
une cellule interne (120) comprenant une jonction d'ancrage (124) accouplée à l'extrémité de sortie de la cellule externe, et dans lequel la cellule interne comprend une jonction mobile (122) ;
un feuillet (130) comprenant un bord d'entrée et un bord de sortie, dans lequel le bord d'entrée du feuillet est accouplé à la jonction mobile de la cellule interne, et dans lequel le bord de sortie du feuillet est accouplé à la jonction d'ancrage ; et
une lumière (350a) traversant le limiteur de débit, dans lequel la lumière est définie au moins en partie par le feuillet,
dans lequel la jonction mobile de la cellule interne et le bord d'entrée du feuillet sont configurés pour se déplacer axialement en réponse à une première augmentation de la pression artérielle à l'intérieur du vaisseau sanguin, et en réponse au fait que la pression artérielle à l'intérieur du vaisseau sanguin se trouve à l'intérieur d'une première plage de pression artérielle, dans lequel le feuillet est incurvé et est configuré pour fléchir en réponse au fait que la jonction mobile se déplace axialement, et
dans lequel la jonction mobile et le feuillet sont configurés de telle sorte que la lumière change de taille lorsque la jonction mobile se déplace.

2. Limiteur de débit selon la revendication 1, comprenant en outre une seconde cellule externe, une seconde cellule interne comprenant une seconde jonction mobile, et un second feuillet, dans lequel un bord d'entrée du second feuillet est accouplé à la seconde jonction mobile.

3. Limiteur de débit selon la revendication 2, comprenant en outre deux cellules externes ou plus, deux cellules internes ou plus comprenant chacune une jonction mobile, et deux feuillets ou plus, dans lequel un bord d'entrée de chacun des deux feuillets ou plus est accouplé à une jonction mobile,
éventuellement dans lequel les deux feuillets ou plus sont accouplés ensemble et les bords d'entrée des deux feuillets ou plus forment un bord festonné.

4. Limiteur de débit selon une quelconque revendication précédente, comprenant en outre un fil conducteur, dans lequel la jonction mobile est accouplée au fil conducteur, dans lequel la jonction mobile est configurée pour se déplacer axialement d'une première position à une deuxième position en réponse à un premier courant électrique appliqué au fil conducteur, dans lequel la deuxième position est plus proche de l'extrémité de sortie de la cellule externe que la première position, et dans lequel le feuillet est configuré pour fléchir de telle sorte que la lumière soit plus faible lorsque la jonction mobile est dans la deuxième position que lorsque la jonction mobile est dans la première position, éventuellement dans lequel le fil conducteur est :
a) configuré pour changer de longueur ou changer de forme en réponse au premier courant électrique ; ou
b) un alliage à mémoire de forme, ou du nitinol.

5. Limiteur de débit selon la revendication 4, comprenant en outre un capteur configuré pour mesurer la pression artérielle à l'intérieur du vaisseau sanguin, dans lequel le capteur est une jauge de contrainte, un capteur piézoélectrique, un capteur capacitif ou un capteur de pression à vide, et dans lequel le premier courant électrique est appliqué en réponse à un signal provenant du capteur.

6. Limiteur de débit selon la revendication 4, dans lequel la jonction mobile est configurée pour se déplacer axialement de la deuxième position à une troisième position en réponse à un second courant électrique appliqué au fil conducteur, dans lequel la troisième position est plus proche de l'extrémité de sortie de la cellule externe que la deuxième position, dans lequel le feuillet est configuré pour entrer en prolapsus ou s'affaisser lorsque la jonction mobile est dans la troisième position de telle sorte que la lumière soit plus grande lorsque la jonction mobile est dans la troisième position qu'elle ne l'est lorsque la jonction mobile est dans la deuxième position.

7. Limiteur de débit selon la revendication 6, dans lequel le second courant électrique est appliqué en réponse à une seconde augmentation de la pression artérielle à l'intérieur du vaisseau sanguin, et en réponse au fait que la pression artérielle à l'intérieur du vaisseau sanguin est à l'intérieur d'une seconde plage de pression artérielle, dans lequel une limite inférieure de la seconde plage de pression artérielle est supérieure à une limite supérieure de la première plage de pression artérielle.

8. Limiteur de débit selon la revendication 7, dans lequel la première plage de pression artérielle est associée à une surcharge volumique de pression artérielle, et la seconde plage de pression artérielle est associée à une pression artérielle liée à l'effort.

9. Limiteur de débit selon la revendication 7 ou la revendication 8, comprenant en outre un capteur configuré pour mesurer la pression artérielle à l'intérieur du vaisseau sanguin, dans lequel le capteur est une jauge de contrainte, un capteur piézoélectrique, un capteur de capacité ou un capteur de pression à vide, et dans lequel le premier courant électrique et le second courant électrique sont appliqués en réponse à des signaux provenant du capteur.

10. Limiteur de débit selon une quelconque revendication précédente, dans lequel l'écoulement sanguin à l'intérieur du vaisseau sanguin peut entrer en contact avec une surface extérieure (232) du feuillet, ou une surface intérieure (234) et la surface extérieure (232) du feuillet, dans lequel chaque jonction mobile est configurée pour se déplacer axialement d'une première position à une seconde position en réponse au fait que la pression artérielle applique une première pression à la surface extérieure ou à la surface intérieure et à la surface extérieure du feuillet, dans lequel la seconde position est plus proche de l'extrémité de sortie de la cellule extérieure que la première position, et dans lequel la lumière est plus petite lorsque la jonction mobile est dans la deuxième position que lorsque la jonction mobile est dans la première position.

11. Limiteur de débit selon la revendication 10, dans lequel la jonction mobile est configurée pour se déplacer axialement de la deuxième position à une troisième position en réponse au fait que la pression artérielle applique une seconde pression à la surface extérieure ou à la surface intérieure et à la surface extérieure du feuillet, et en réponse au fait que la pression artérielle à l'intérieur du vaisseau sanguin se trouve dans une deuxième plage de pression artérielle, dans lequel une limite inférieure de la deuxième plage de pression artérielle est supérieure à une limite supérieure de la première plage de pression artérielle, dans lequel la troisième position est plus proche de l'extrémité de sortie de la cellule externe que la deuxième position, dans lequel le feuillet est configuré pour entrer en prolapsus ou s'affaisser lorsque la jonction mobile est dans la troisième position, et dans lequel la lumière est plus grande lorsque la jonction mobile est dans la troisième position qu'elle ne l'est lorsque la jonction mobile est dans la deuxième position.

12. Limiteur de débit selon la revendication 11, dans lequel la première plage de pression artérielle est associée à une surcharge volumique de pression artérielle, et la seconde plage de pression artérielle est associée à une pression artérielle liée à l'effort, éventuellement dans lequel la première plage de pression artérielle va de 10 mmHg à 25 mmHg et la seconde plage de pression artérielle est supérieure à environ 25 mmHg ou supérieure à environ 30 mmHg.

13. Limiteur de débit selon la revendication 10, dans lequel la cellule interne est configurée pour changer de longueur ou changer de forme en réponse à la première pression.

14. Limiteur de débit selon une quelconque revendication précédente, dans lequel le vaisseau sanguin est une veine cave inférieure ou une veine en amont d'une veine rénale.

15. Limiteur de débit selon une quelconque revendication précédente, dans lequel la jonction mobile est déplacée en utilisant un élément de commande (140) ou un fil de traction accouplé à un mécanisme de commande actif, éventuellement dans lequel le mécanisme de commande actif comprend l'un ou plusieurs parmi : un microcontrôleur, une batterie, un câble d'alimentation de prise murale, un système de communication avec ou sans fil, un système d'induction de champ magnétique, un système d'induction de champ électromagnétique, un système de polarisation magnétique, un actionneur linéaire, un capteur, un système à piston, un système à vanne, un montage hydraulique, un montage pneumatique, des actionneurs optiques, des actionneurs thermiques, et d'autres actionneurs utilisant une alimentation électrique ou inductive.
